(19) Europäisches Patentamt
European Patent Office
Office européen des brevets

(11) Publication number: 0 258 476 B1

(12) # EUROPEAN PATENT SPECIFICATION

(45) Date of publication of the patent specification: 25.04.90

(51) Int. Cl.[4]: **A61K 31/40**, A61K 31/415, A61K 31/42, A61K 31/425, A61K 31/44, A61K 31/50, A61K 31/505, A61K 31/41

(21) Application number: 86112137.4

(22) Date of filing: 02.09.86

(54) **Aldose reductase inhibitors useful in glaucoma therapy.**

(43) Date of publication of application: 09.03.88 Bulletin 88/10

(45) Publication of the grant of the patent: 25.04.90 Bulletin 90/17

(84) Designated Contracting States: AT BE CH DE FR GB IT LI LU NL SE

(56) References cited:
EP-A- 0 136 143
US-A- 4 609 663

Diabetes vol. 35 no.4 April 1986 pp 426-432. Barnett et al "The effect of oxidation on sorbitol pathway kinetics"
Chem. Pharm. Bull. vol. 33 no. 7 1985 pp 2990-2995. Okuda et al "Effects of an aldose reductase inhibitor , 1( (p-bromophenyl)-sulfony) hydantoin, on cataract formation and tissue polyol levels in galactosemic rats

The file contains technical information submitted after the application was filed and not included in this specification

(73) Proprietor: ALCON LABORATORIES INC, 6201 South Freeway P.O. Box 1959, Fort Worth Texas 76101(US)

(72) Inventor: York, Billie Murray, Jr., 5922 South Westcreek Court, Fort Worth Texas 76133(US)

(74) Representative: TER MEER - MÜLLER - STEINMEISTER & PARTNER, Mauerkircherstrasse 45, D-8000 München 80(DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

Background of the Invention

This invention relates to the use of aldose reductase inhibitors for the manufacture of a medicament for treating the progressive field vision loss associated with primary open angle glaucoma. Specifically, this invention relates to the preparation of compositions comprising an aldose reductase inhibitor for this medical indication. The administration of these compositions to treat the glaucoma syndrome, a leading cause of blindness is illustrated in this context.

While Applicant is bound by no theory, it appears that an important mechanism underlying open angle glaucoma is one associated with the enzyme aldose reductase and/or related aldehyde reductases. Certain cells of the trabecular meshwork, lamina cribrosa and lamina vasculosa of the eye contain the enzyme aldose reductase and/or related aldehyde reductases. These reductases under conditions of hyperglycemia or hypergalactosemia cause the accumulation of certain polyols such as sorbitol or galactitol respectively. The inhibition of the enzyme aldose reductase and related reductases results in the retardation of abnormal polyol accumulation at the expense of NADPH in such ocular cells. Such inhibition preserves normal or near normal reduced glutathione status, $Na^+/K^+$ ATPase activity and amino acid transport within said cells. Furthermore, this inhibition promotes or preserves normal collagen production in and around these cells of the trabecular meshwork which are susceptible to high aldose concentrations. This includes cells of the lamina cribrosa and lamina vasculosa in mammalian eyes, particularly those of man. Trabecular meshwork cells are important in preserving proper aqueous humor outflow facility of the eye. Intraocular pressure or tension in part corresponds to outflow facility. In certain conditions of elevated intraocular pressure, the outflow of the trabecular meshwork may be impaired due to abnormal collagen metabolism and insufficient trabecular meshwork cell population. The rheological characteristics of the trabecular meshwork are linked to the normal functions of its cells.

Pathological alterations in collagen production and support in the posterior segments of the eye, which include the lamina cribrosa and lamina vasculosa, are associated with impaired retinal function and vision loss in glaucoma. This weakening of the posterior support structures of the eye is suggested to arise from abnormally elaborated collagen. Aldose reductase inhibitors, such as those disclosed in the present invention, have been demonstrated to prevent an abnormal elaboration of collagen associated with hyperglycemia and/or hypergalactosemia. Hyperglycemia is believed to be associated with the compositions of glaucoma in diabetes mellitus patients. It follows that moderate glucose intolerance as is associated with aging may be contributory to glaucoma. Therefore, a potent aldose reductase inhibitor will have therapeutic utility as an agent in the therapy and/or prevention of glaucoma.

In this context, especially with regard to the so-called cataract which is different from the above glaucoma type, the prior art documents Chem. Pharm. Bull. 33 (1985), pgs. 2990–2995, and Diabetes, 35 (Apr. 1986), pgs. 426–432 are of interest.

Thus, it is an object of the present invention to provide the aldose reductase inhibitors for the above use to produce pharmaceutical compositions which will retard or delay the progressive field of vision loss associated with glaucoma.

Detailed Description of the Invention

Aldose reductase inhibitors which are particularly suitable for the method of the present invention and pharmaceutical compositions comprising these inhibitors are disclosed in the prior patent art, many of the patents being owned by the Applicant. Preferred aldose reductase inhibitors which may be used in the invention are disclosed in Applicant's U.S. Patent Nos., i.e. US-A 4 537 892 and 4 436 745. Also disclosing suitable aldose reductase inhibitors for use in the invention are U.S. Patents, i.e. US-A 4 438 272, - 3 821 383, - 4 117 230, - 4 130 714 and 4 181 782. U.S. Patent No., i.e. US-A 4 537 892 corresponds to EP-A 137 333, filed September 12, 1984.

It should be understood that the invention is inclusive of any aldose reductase inhibitor which is useful in the treatment of glaucoma and particularly, primary open angle glaucoma. Classes of aldose reductase inhibitors which may be used in the present invention include spirocyclic aromatic imides (U.S. Patent, i.e. US-A 4 537 892); spirohydantoin compounds (U.S. Patent Nos., i.e. US-A 4 130 714, - 4 117 230, - 4 436 745, - 4 438 272 and 4 209 630); spiro-oxazolidinediones (U.S. Patent, i.e. US-A 4 226 875) and spiro-polycyclicimidanzolidinediones (U.S. Patent, i.e. US-A 4,181,728), as well as other aldose reductase inhibitors known in the art.

A particularly preferred group of aldose reductase inhibitors useful in the invention are the spirocyclic aromatic imides described in U.S. Patent No., i.e. US-A 4 537 892 which are of the following general formula:

I. 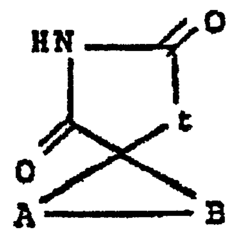

and the pharmaceutically acceptable metal salts and in cases, where basic aromatic nitrogens are in the A, and/or B rings, the pharmaceutically acceptable organic and inorganic acid salts thereof, wherein A and B are aromatic or heterocyclic rings connected through two adjacent positions to a central cycloalkyl ring, the A and B rings being selected from the group consisting of those of the formula:

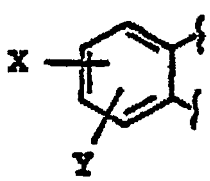 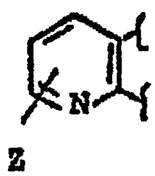 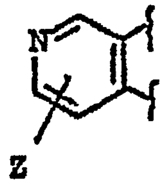 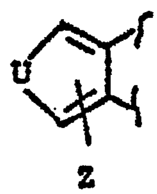 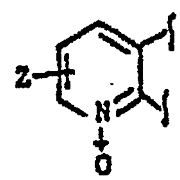

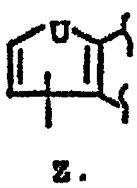 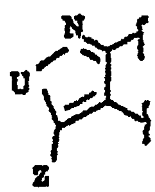 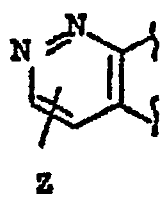 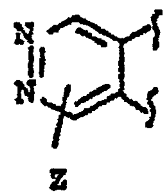 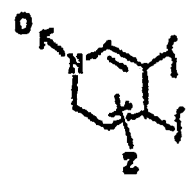

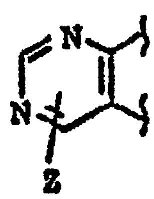 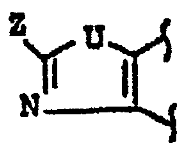 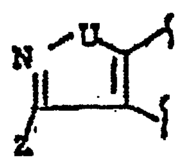

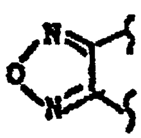 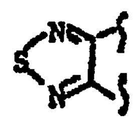

and wherein U is selected from the group consisting of O, S, N-$R^1$;
X is selected from the group consisting of H, F, lower alkyl sulfide (e.g., -S-$CH_3$), lower alkylsulfinyl (e.g., -S(O)$CH_3$);
Y is selected from the group consisting of H, -OH, and

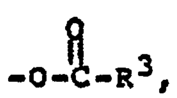

F, Cl, lower alkyl, lower alkoxy, lower alkylsulfide (e.g., -S-$CH_3$), lower alkyl-sulfinyl (e.g., -S(O)-$CH_3$), lower alkylsulfonyl (e.g., -$SO_2CH_3$), -$CF_3$, -S-$CF_3$, -$SO_2CF_3$, CO-N($R^1$)-$R^2$, lower alkyl alcohol (e.g., -$CH_2$-OH), lower alkyl ether (e.g., -$CH_2OCH_3$), nitro, lower alkyl sulfide lower alkyl (e.g., -$CH_2$S- $CH_3$), lower alkylamine (e.g., -$CH_2NH_2$), lower alkyl esters (e.g., -$CH_2$-O-$COCH_3$), carboxylic acids and lower alkyl esters (e.g., -$COOR^3$), lower alkyl carboxylic acids and esters (e.g., -CH($CH_3$)-$COOR^1$), and lower cycloalkyl (e.g., cyclopropyl);

$R^1$ and $R^2$ are selected from the group consisting of H and lower alkyl (preferably methyl or ethyl);
$R^3$ is lower alkyl (preferably methyl or ethyl);
Z is selected from the group consisting of H, lower alkyl (preferably methyl), and halogen (fluoro, chloro, bromo, iodo); and
t is selected from the group consisting of NH, O, S, and $CHR^1$.

In a more preferred embodiment, the spiro-cyclic aromatic imides of the present invention are of the following general formula:

I-A. 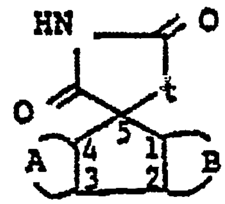

wherein A, B, U, X, Y, $R^1$, $R^2$, $R^3$, Z and t are as described above. In more preferred embodiments, the cycloalkyl groups have 4 to 7 carbon atoms and lower alkyl groups have 1 to 6 carbon atoms. In an especially preferred emodiment, Ring A is selected from the foregoing group and Ring B is selected from the group consisting of the following:

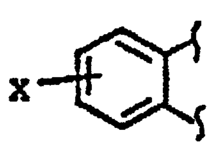 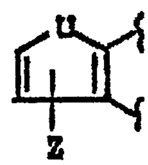 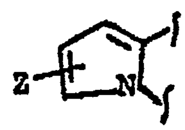 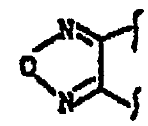

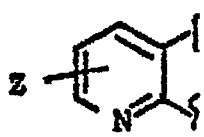 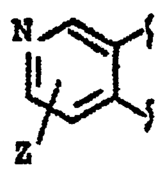 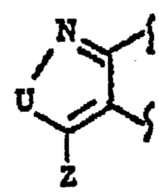 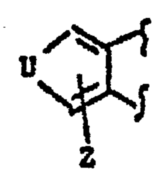

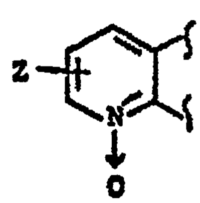 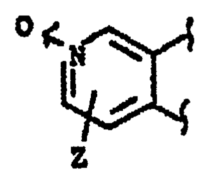 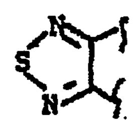

wherein X, U, and Z are described above. In the compounds of Formulae I and I-A, Rings A and B are attached to the central five-membered ring at positions 1,2 and 3,4.

The compounds of this type have important geometric and chemical similarities. These similarities include a planar, rigid tricyclic fluorene or fluorene-like aromatic ring system spiro-coupled to a five-membered imide (or cyclic secondary amide) ring such as succinimide, hydantoin, thiazolidinedione or oxazolidinedione. These spirocyclic derivatives of the various tricycles each contain a polarizable and hydrogen-bondable secondary amide, also called imide, radical (-CO-NH-CO-).

In those instances where, according to general Formulae I and I-A, A does not equal B, the spiro carbon is chiral. Activity of any such racemic mixture may be attributable to only one isomer. Resolution, or direct synthesis, of the enantiomers, as is known in the art, is recognized as a method to isolate or prepare the active or the more active enantiomer. It is also recognized that certain patterns of substitution on A and/or B according to Formulae I and I-A may create asymmetry, and the resulting diastereomeric mixtures may be separated by chromatography or solvent recrystallizations, as is known and practiced in the art. For example, if A has a methylsulfoxyl substituent and A is different from B, then there are at least two chiral centers: the spiro carbon and the sulfoxide sulfur. Physical separation of this diastereomeric mixture by chromatography or other methods practiced in the art will yield two racemic mixtures, each containing a pair of enantiomers. Stereospecific oxidation of a methylsulfide on A to yield a methyl-

sulfoxide (e.g., via sodium metaperiodate and albumin) when A is different than B (according to Formula I) will yield a diastereomeric mixture, which then can be separated by conventional physical methods known in the art, such as liquid chromatography or differential solvent solubility, to yield the purified diastereomers which are themselves purified optical isomers. Reduction of the two optically active sulfoxide diastereomers will yield the optically active pair of enantiomers of mirror image isomers.

Other useful aldose reductase inhibitors to be used in the invention are disclosed in the following patents and patent publications;

1. European Patent Application No. EP-A 180 421, Pfizer, Inc.
2. Japanese Patent Application No. J- 61 056 175-A, Yamanouchi Pharm. KK
3. European Patent Application No. EP 173 421, Pfizer, Inc.
4. European Patent Application No. EP-A 172 719, Pfizer, Inc.
5. European Patent Application No. EP-A 171 213, Pfizer, Inc.
6. European Patent Application No. EP-A 159 143, Pfizer, Inc.
7. U.S. Patent No. i.e. US-A 4 540 700, Alcon Labs, Inc.
8. U.S. Patent No. i.e. US-A 4 533 667, Pfizer, Inc.
9. U.S. Patent No. i.e. US-A 4 593 092, Kyorin Pharm. Ltd.
10. Canadian Patent No. i.e. CA-A 1 187 093, Ayerst Mc Kenna & HA
11. European Patent Application No. EP-A 143 461, Kaken Pharm. Co. Ltd.
12. European Patent Application No. EP-A 136 143, Pfizer, Inc.
13. U.S. Patent No. i.e. US-A 4 587 256, Hoffmann-La Roche AG
14. French Patent No. 2 544 317, ADIR
15. U.S. Patent No. i.e. US-A 4 471 124, Pfizer, Inc.
16. European Patent Application No. EP-A 117 035, Pfizer, Inc.
17. European Patent Application No. EP-A 127 412, Pfizer, Inc.
18. European Patent Application No. EP-A 115 133, Pfizer, inc
19. U.S. Patent No. i.e. US-A 4 457 941, Syntex, Inc.
20. European Patent Application No. EP-A 112 620-A, Pfizer, Inc.
21. U.S. Patent No. i.e. US-A 4 452 806, Pfizer, Inc.
22. U.S. Patent No. 4 442 118, i.e. US-A Ayerst McKenna Corp.
23. European Patent Application No. EP-A 104 078-A, Ayerst McKenna Corp.
24. Canadian Patent No. 1 162 856, Ayerst McKenna & HA.
25. U.S. Patent No. i.e. US-A 4 585 789, Fujisawa Pharm. KK.
26. German Patent Application DE-A 3 315 106, ADIR.
27. U.S. Patent No. i.e. US-A 4 464 385, Alcon Labs, Inc.
28. European Patent Application No. EP-A 91 761, Takeda Chem. Ind. KK
29. U.S. Patent No. i.e. US-A 4 443 465, ICI
30. U.S. Patent No. i.e. US-A 4 490 381, ICI.

To the extent that these applications and patents disclose aldose reductase inhibitors which are useful in the practice of the present invention, they are cited herein to illustrate the invention.

The following specific compounds and their optical isomers are specifically preferred aldose reductase inhibitors for use in the invention:

Spiro-(7-fluore-4H-indeno[1,2-b] thiophen-4,4'-imidazolidine)-2',5'-dione;
Spiro-(2-fluore-9H-fluoren-9,4'-imidazolidine)-2',5'-dione
Spiro-(2-fluoro-9H-fluoren-9,3'-succinimide);
Spiro-(7-fluoro-5H-indeno[1,2-b]-pyridin-5,4'-imidazolidine)-2',5'-dione;
Spiro-(7-chloro-5H-indeno[1,2-a]pyridin-9,4'-imidazolidine)-2',5'-dione;
Spiro-(7-chloro-9H-pyrrolo[1,2-a]indol-9,4'-imidazolidine)-2',5'-dione;
Spiro-(2,7-difluoro-9H-fluoren-9,4'-imidazolidine)-2',5'-dione;
Spiro-(2,7-difluoro-9H-fluoren-9,3'-succinimide);
Spiro-(2,7-difluoro-9H-fluoren-9,5'-oxazolidine)-2',4'-dione;
Spiro-(7-fluoro-5H-indeno-[1,2-b]pyridine-5,3'-succinimide);
Spiro-(7-chloro-5H-indeno-[1,2-b]pyridine-5,3'-succinimide);
Spiro-(7-methylthio-5H-indeno-[1,2-b]pyridine-5,3'-succinimide);

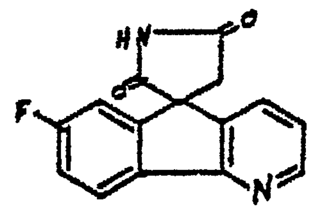

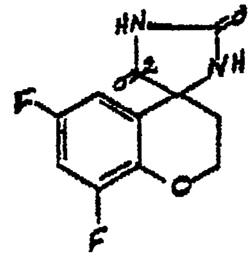

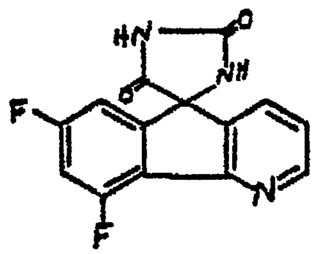

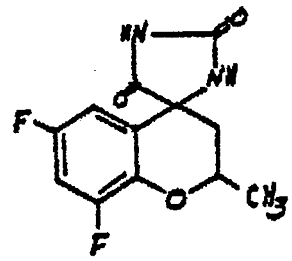

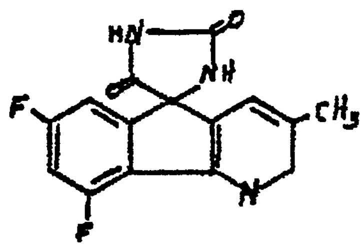

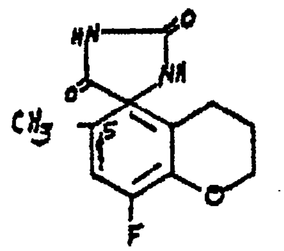

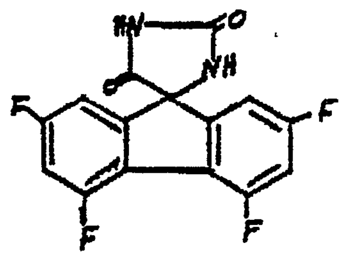
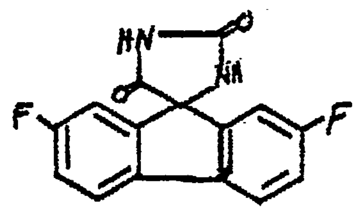
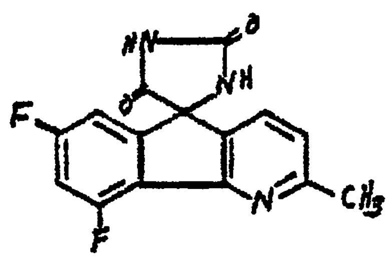
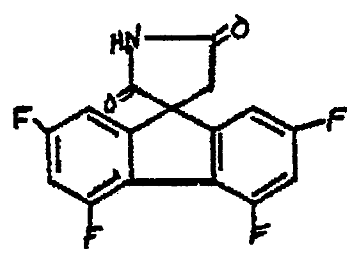
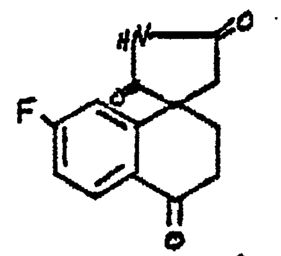
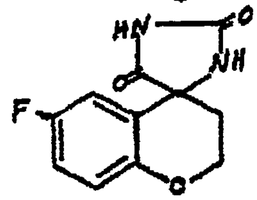
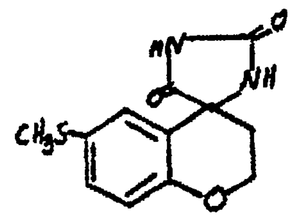
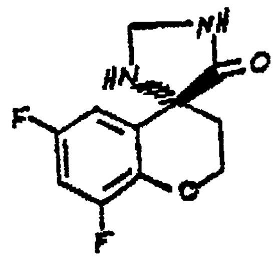
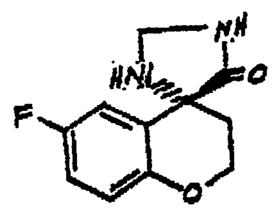

The preferred route of administration for the compositions of the present invention is topically to the eye or per os. The exact dosage regimen is left to the routine discretion of clinician taking into consideration the patient's age, sex, weight, and his history accounting for or attributing to the glaucoma in question.

The most preferred compositions will have the aldose reductase inhibitor of choice present at a concentration ranging from 0.1% to 2.0 weight % in a vehicle selected from buffered water, aqueous buffered carbopol® gel, which is a carboxypolymethylene preparation or a perfluoroalkane-type vehicle such as a fluorocarbon.

Typical systemic routes of administration are fully disclosed and claimed in copending owned U.S. Patent No. i.e. US-A 4 436 745 and i.e. US-A 4 537 892, which are discussed above.

The following representative examples illustrate the manufacture of the suitable pharmaceutical compositions for topical or oral delivery of the involved aldose reductase inhibitors for glaucoma therapy.

Example 1

Gel composition for topical, ocular administration:

| Ingredient | % by weight |
|---|---|
| 0.25% w/v of the compound | 0.25% |
| Spiro-(7-chloro-5Hindeno([1,2-b] -pyridin-5,4'-imidazolidine)-2',5'-dione | |
| Benzalkonium Chloride | 0.01% |
| Carboxypolymethylene (Carbopol®) | |
| Hydrochloric Acid and/or | to adjust pH |
| Sodium hydroxide | to 5.0 to 5.5 |
| Purified Water (as gel) | q.s. to 100% |

The following topical, ocular formulations are physically in the form of suspensions:

Suspension A

| Ingredient | % by weight |
|---|---|
| Micronized Spiro-(2-fluoro-9H-fluoren-9,4'-imidazolidine)-2',5-dione | 1.0% |
| Perfluorotributylamine | 99.0% |

Suspension B

| Suspension B | |
|---|---|
| Ingredient | % by weight |
| Micronized Spiro-(2,7-difluoro-9H-fluoren-9,3'-succinimide) | 1.0 |
| Hydroxymethylcellulose | 1.0% |
| Disodium edetate | 0.01% |
| Benzalkonium chloride | 0.01% |
| Sodium Acetate | 0.14% |
| Sodium Chloride | 0.52% |
| Hydrochloric Acid and/or Sodium hydroxide | ph 4.5 to 5.5 |
| Purified Water (as suspension) | q.s. to 100% |

The following formulation is a selected representative of a solution for the ophthalmic indications of the present invention:

| Ingredient | % by weight |
|---|---|
| Spiro-(7-fluoro-5H-indeno [1,2-b] pyridine-5,3'-succinimide) | 0.10% |
| Carboxypolymethylene (Carbopol®) | 0.10% |
| Benzalkonium Chloride | 0.008% |
| Hydrochloric Acid and/or | to adjust |
| Sodium Hydroxide | ph 4.5 to 5.0 |
| Purified Water | q.s. to 100% |

Example 2

A dry solid pharmaceutical composition is prepared by mixing the following materials together in the proportions by weight specified:

| Ingredient | % by weight |
|---|---|
| Micronized Spiro-(2,-7-difluoro-9Hfluoren-9,4'-imidazolidine)-2',5'-dione | 50% |
| Sodium Citrate | 20% |
| Alginic Acid | 5% |
| Polyvinylpyrrolidone | 15% |
| Magnesium Stearate | 5% |

The dry composition is thoroughly blended, tablets are punched from the resulting mixture, each tablet being of such size that it contains 100 mg of the active ingredient. Other tablets are also prepared in a likewise manner containing 10, 25 and 200 mg of active ingredient, respectively, by merely using an appropriate quantity by weight of the spiro-hydantoin in each case. Likewise other related examples of spiro-imidazolidinedions, spiro-thiazolidinediones, spiro-oxazolidinediones, spiro-succinimides can be formulated as tablets on a respective weight proportion.

The dry solid pharmaceutical composition is prepared by combining the following materials together in the weight proportions indicated below:

| Ingredient | % by weight |
|---|---|
| Spiro-(7-fluoro-5H-indeno [1,2-b]pyridin-5,4'-imidazolidine)-2',5'-dione | 50% |
| Calcium Carbonate | 20% |
| Polyethylene glycol, Average Molecular Weight 8,000 | 30% |

The dry solid mixture is thoroughly mixed until uniform in composition. The powdered product is then used to fill soft elastic and hard-gelatin capsules so as to provide capsules containing 200 mg of the active ingredient.

**Claims**

1. The use of aldose reductase inhibitors for the manufacture of a medicament for the treatment of progressive field vision loss associated with primary open angle glaucoma.
2. The use according to claim 1 of aldose reductase inhibitors for the manufacture of a medicament for the treatment of ocular hypertension.
3. The use according to claim 1, characterized in that the medicament is in a form to be administered topically, orally or systemically.
4. The use according to claim 3, characterized in that the aldose reductase inhibitor is introduced into a pharmaceutically acceptable vehicle in a concentration ranging from 0.1 to 2.0 weight percent of the vehicle.
5. The use according to claim 4, characterized in that the vehicle is selected from the group consisting of buffered water, aqueous buffered carboxypolymethylene gel and a perfluoroalkane.
6. The use according to claim 1, characterized in that the medicament is manufactured in the form of a gel, suspension, solution, tablet or capsule.
7. The use according to claim 1, characterized in that the aldose reductase inhibitor is a spiro imide compound.
8. The use according to claim 1, characterized in that the aldose reductase inhibitor is a spirocyclic aromatic imide, a spirohydantoin, a spirooxazolidinedione, or a spiropolycyclicimidazolidinedione.
9. The use according to claim 1, characterized in that the aldose reductase inhibitor is spiro-(2,7-difluoro-9H-fluoren-9,4'-imidazolidine)-2', 5'-dione of the formula

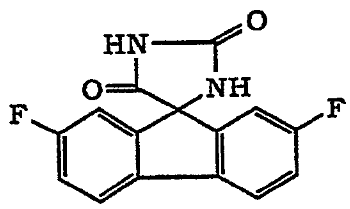

## Patentansprüche

1. Verwendung von Aldosereduktase-Inhibitoren zur Herstellung eines Medikaments zur Behandlung von mit primärem Weitwinkelglaukom begleiteten fortschreitendem Gesichtsfeldverlust.
2. Verwendung von Aldosereduktase-Inhibitoren nach Anspruch 1 zur Herstellung eines Medikaments zur Behandlung von okularem Hypertension.
3. Verwendung nach Anspruch 1, dadurch gekennzeichnet, daß das Medikament in einer Form zur topischen, oralen oder systemischen Verabreichung vorliegt.
4. Verwendung nach Anspruch 3, dadurch gekennzeichnet, daß der Aldosereduktase-Inhibitor in ein pharmazeutisch annehmbares Trägermaterial in einer Konzentration im Bereich von 0,1 bis 2,0 Gew.-% des Trägermaterials eingebracht worden ist.
5. Verwendung nach Anspruch 4, dadurch gekennzeichnet daß das Trägermaterial aus der Gruppe gepuffertes Wasser, wäßriges gepuffertes Carboxypolymethylengel und ein Perfluoralkan gewählt ist.
6. Verwendung nach Anspruch 1, dadurch gekennzeichnet, daß das Medikament in die Form eines Gels, einer Suspension, einer Lösung, einer Tablette oder einer Kapsel gebracht ist.
7. Verwendung nach Anspruch 1, dadurch gekennzeichnet, daß der Aldosereduktase-Inhibitor eine Spiroimidverbindung ist.
8. Verwendung nach Anspruch 1, dadurch gekennzeichnet, daß der Aldosereduktase-Inhibitor ein spirocyclisches aromatische Imid, ein Spriohydantoin, ein Spirooxazolidindion oder ein Spiropolycycloimidazolidindion ist.
9. Verwendung nach Anspruch 1, dadurch gekennzeichnet, daß der Aldosereduktase-Inhibitor die Verbindung Spiro-(2,7-difluor-9H-fluoren9,4′-imidazolidin)-2,5′-dion der Formel

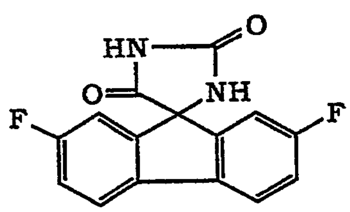

## Revendications

1. Utilisation des inhibiteurs d'aldose réductase pour la fabrication d'un médicament pour le traitement d'un perte progressive du champ visuel associée à un glaucome à angle ouvert primaire.
2. Utilisation selon la revendication 1, des inhibiteurs d'aldose réductase pour la fabrication d'un médicament pour le traitement de l'hypertension oculaire.
3. Utilisation selon la revendication 1, caractérisée en ce que le médicament se présente sous une forme d'administration topique, orale ou systémique.
4. Utilisation selon la revendication 3, caractérisée en ce que l'on introduit l'inhibiteur d'aldose réductase dans un véhicule phamaceutiquement acceptable à une concentration allant de 0,1 à 2,0% en poids du véhicule.
5. Utilisation selon la revendication 4, caractérisée en ce que le véhicule est choisi dans le groupe comprenant l'eau tamponnée, le gel de carboxypolyméthylène tamponné aqueux, et un perfluoroalcane.
6. Utilisation selon la revendication 1, caractérisée en ce que le médicament est fabriqueé sous forme d'un gel, d'une suspension, d'une solution, d'un comprimé ou d'une gélule.
7. Utilisation selon la revendication 1, caractérisée en ce que l'inhibiteur d'aldose réductase est un composé spiroimide.
8. Utilisation selon la revendication 1, caractérisée en ce que l'inhibiteur d'aldose réductase est un imide de aromatique spirocyclique, une spirohydantoïne, une spirooxazolidinedione, ou une spiropolycycloimidazolidinedione.
9. Utilisation selon la revendication 1, caractérisée en ce que l'inhibiteur d'aldose réductase est la spiro-(2,7-difluoro-9H-fluorène-9,4′-imidazolidine)-2′,5′-dione de formule